# EUROPEAN PATENT SPECIFICATION

(11) **EP 4 017 461 B1**
(45) Date of publication and mention of the grant of the patent: **08.07.2026**
(21) Application number: 20824877.3
(22) Date of filing: 20.11.2020
(51) Int. Cl.: A61K 8/27, A61K 8/44, A61K 8/60, A61Q 11/00

(54) **VISCOSITY STABLE SLS FREE TOOTHPASTES CONTAINING ZINC COMPOUNDS AND ARGININE**
VISKOSITÄTSSTABILE SLS-FREIE ZAHNPASTEN MIT ZINKVERBINDUNGEN UND ARGININ
PÂTES DENTIFRICES EXEMPTES DE SLS STABLES À LA VISCOSITÉ CONTENANT DES COMPOSÉS DE ZINC ET DE L'ARGININE

(30) Priority: 10.12.2019 US 201962946116 P
(43) Date of publication of application: 29.06.2022
(73) Proprietor: Colgate-Palmolive Company, New York, NY 10022 (US)
(72) Inventor: POTH, Tilo, 69469 Weinheim (DE)
(74) Representative: Sonnenhauser, Thomas Martin
(86) International application number: PCT/US2020/070805
(87) International publication number: WO 2021/119650

(56) References cited:
- US-A1- 2015 297 500
- US-A1- 2019 175 478
- US-A1- 2019 307 657
- DATABASE GNPD [online] MINTEL; 25 November 2019 (2019-11-25), ANONYMOUS: "Complete Care Natural Toothpaste", XP055778636, retrieved from https://www.gnpd.com/sinatra/recordpage/7058675/ Database accession no. 7058675
- DATABASE GNPD [online] MINTEL; 15 May 2018 (2018-05-15), ANONYMOUS: "Biocare Toothpaste", XP055703101, retrieved from https://www.gnpd.com/sinatra/recordpage/5669395/ Database accession no. 5669395
- N. LOURITH ET AL: "Natural surfactants used in cosmetics: glycolipids", INTERNATIONAL JOURNAL OF COSMETIC SCIENCE., vol. 31, no. 4, 1 August 2009 (2009-08-01), NL, pages 255 - 261, XP055371172, ISSN: 0142-5463, DOI: 10.1111/j.1468-2494.2009.00493.x

## Description

### BACKGROUND

Arginine-based oral care compositions generally include some combination of polymers, abrasive(s), and in some instances, additional active ingredients. Zinc compounds are fairly common ingredients for use in oral care compositions. In these products, zinc compounds are utilized as an antimicrobial ingredient to prevent gum inflammation. Commonly used zinc compounds are zinc citrate, zinc lactate, zinc oxide, and zinc nitrate. In those instances where additional active ingredients are included and comprise cationic metal ions, e.g., zinc, maintaining the physical stability of the composition is a challenge because of the interaction between these cationic metal ions and certain polymeric components and abrasive systems.

Formulating toothpastes with free zinc ions typically suffers from incompatibility with ionic thickeners or binders like carboxymethyl cellulose (CMC) or xanthan gum. These polymers react with zinc ions, leading to a drop in viscosity and sometimes to the formation of lumps and undesirable separation. Maintaining the formula viscosity within a certain range is critical because viscosity impacts physical stability as well as processability. Alternative thickeners like hydroxyethyl cellulose (HEC) do not often yield desired rheological properties, leaving a toothpaste with non-optimal stand-up that sinks through the bristles of a toothbrush. Further disadvantages that can be linked to the use of HEC are gummy paste properties, impaired pumping properties of the product (loss of sheer thinning), high cost and relative low availability. For these reasons, alternative gum systems have so far not been found to provide a satisfactory technical solution of the problem.

Typically zinc is a foam breaking ingredient. As foam formation is essential for both product performance (cleaning properties) and consumer acceptance, conventional toothpastes contain a high level of surfactants, particularly sodium lauryl sulfate (SLS), often at concentrations above 2 %. However, consumer studies and surveys have shown that there is a desire to use oral care compositions that do not contain sodium lauryl sulfate, as some consumers experience relatively greater sensitivity to this ingredient.

As such, there remains a need in the art for oral care compositions with stable viscosity as well as good foaming properties. US 2015/0297500 A1 discloses oral care compositions comprising an orally acceptable vehicle, a basic amino acid in free or salt form, particles of precipitated calcium carbonate, a source of zinc ions, and a surfactant system selected from at least one of a poloxamer nonionic surfactant and a betaine zwitterionic surfactant or a mixture thereof.

### BRIEF SUMMARY

In one aspect, the invention provides an oral care composition, e.g., toothpaste or gel, which comprises a basic amino acid in free or salt form, wherein the basic amino acid comprises arginine and is present in an amount from 1% to 15% by weight of the composition, being calculated as free base form; a zinc ion source and a surfactant system comprising lauryl glucoside and sodium cocoyl glutamate; or a glycolipid; wherein lauryl glucoside, if present, is present in an amount of from 2% to 3% by weight of the composition and sodium cocoyl glutamate, if present, is present in an amount of from 2% to 3% by weight of the composition; wherein the glycolipid, if present, is present in an amount of from 3% to 7% by weight of the composition; and wherein the composition is free of sodium lauryl sulfate as defined in the claims. The composition is free of sodium lauryl sulfate. The surfactant system comprises lauryl glucoside and sodium cocoyl glutamate; or a glycolipid as further defined in the claims. Generally, an alkyl glucoside may be C₈₋₂₅ alkyl glucoside, e.g., C₈₋₁₈ alkyl glucoside, or C₁₀₋₁₈ alkyl glucoside, e.g., lauryl glucoside. Lauryl glucoside , if present, is present in an amount of from 2% to 3% as defined in the claims, or from 2.2% to 2.8%, from 2.4% to 2.6%, or about 2.5% by weight of the composition. Generally, an acyl glutamate may be C₈₋₂₅ acyl glutamate, e.g., C₈₋₁₈ acyl glutamate, or C₁₀₋₁₈ acyl glutamate, e.g., sodium cocoyl glutamate. Generally, acyl glutamate may be present in an amount of from 2% to 3%, from 2.2% to 2.8%, from 2.4% to 2.6%, or about 2.5% by weight of the composition. In certain embodiments, the surfactant system comprises lauryl glucoside and sodium cocoyl glutamate. In other embodiments, the surfactant system comprises a glycolipid. Glycolipid may be rhamnolipid, sophorolipid, trehalolipid, cellobiose lipid, or mannosylerythritol lipid. Glycolipid, if present, is present in an amount of from 3% to 7% as defined in the claims, or from 4% to 6%, from 4% to 5%, or about 4.5% by weight of the composition. In certain embodiments, the surfactant system comprises rhamnolipid.

In some embodiments, the surfactant system further comprises a betaine zwitterionic surfactant, e.g., cocamidopropyl betaine, and a non-ionic block copolymer, e.g., poloxamer 407. The betaine zwitterionic surfactant may be present in an amount of from 1% to 1.5%, from 1.1% to 1.4%, from 1.2% to 1.3%, or about 1.25% by weight of the composition. The non-ionic block copolymer may be present in an amount of from 0.3% to 0.7%, from 0.4% to 0.6%, or about 0.5% by weight of the composition.

The basic amino acid comprises arginine and is present in an amount from 1% to 15% by weight of the composition, being calculated as free base form as defined in the claims. In some embodiments, the basic amino acid further comprises, lysine, citrulline, ornithine, creatine, histidine, diaminobutanoic acid, diaminopropionic acid, salts thereof, and combinations thereof. The basic amino acid comprises arginine and may be present in an amount of e.g., from 1% to 10%, from 1% to 5%, from 1% to 3%, from 1% to 2%, from 1.2% to 1.8%, from 1.4% to 1.6%, or about 1.5% by weight of the composition, being calculated as free base form. In some embodiments, the basic amino acid is arginine.

In some embodiments, the zinc ion source is selected from the group consisting of zinc oxide, zinc sulfate, zinc chloride, zinc citrate, zinc lactate, zinc gluconate, zinc malate, zinc tartrate, zinc carbonate, zinc phosphate and combinations thereof. The zinc ion source may be present in an amount of from 0.01 % to 5 %, e.g., 0.1% to 4%, or 0.5% to 3%, by weight of the composition. In some embodiments, the zinc ion source is selected from zinc oxide, zinc citrate and a combination thereof. In some embodiments, zinc oxide is present in an amount of 0.5 % to 2%, e.g., 0.5% to 1.5%, or about 1% by weight of the composition. In some embodiments, zinc citrate is present in an amount of 0.1%-1%, 0.25-0.75%, about 0.5% by weight of the composition. In certain embodiments, the zinc ion source is a combination of zinc oxide and zinc citrate.

Generally, a method can be comprising applying an effective amount of any of oral care compositions as disclosed herein to the oral cavity, e.g., by brushing, to a subject in need thereof, to (i) reduce or inhibit formation of dental caries, (ii) reduce, repair or inhibit pre-carious lesions of the enamel, (iii) reduce or inhibit demineralization and promote remineralization of the teeth, (iv) reduce hypersensitivity of the teeth, (v) reduce or inhibit gingivitis, (vi) promote healing of sores or cuts in the oral cavity, (vii) reduce levels of acid producing bacteria, (viii) reduce or inhibit microbial biofilm formation in the oral cavity, (ix) reduce or inhibit plaque formation in the oral cavity, (x) promote systemic health, or (xi) clean teeth and oral cavity.

Generally, the use of a surfactant system can be comprising one or more of alkyl glucosides, acyl glutamates, glycolipids, or a combination thereof in the manufacture of an oral care composition, e.g., toothpaste or gel, comprising a basic amino acid in free or salt form and a zinc ion source, e.g., any of oral care compositions as disclosed herein.

Generally, the use of a surfactant system can be comprising one or more of alkyl glucosides, acyl glutamates, glycolipids, or a combination thereof in an oral care composition, e.g., toothpaste or gel, comprising a basic amino acid in free or salt form, and a zinc ion source, e.g., any of oral care compositions as disclosed herein, for stabilizing the viscosity of the composition and increasing the foaming of the composition in an oral cavity when applying the composition to the oral cavity.

Further areas of applicability of the present disclosure will become apparent from the detailed description provided hereinafter. It should be understood that the detailed description and specific examples, while indicating some typical aspects of the disclosure, are intended for purposes of illustration only and are not intended to limit the scope of the disclosure which is defined in the claims.

### BRIEF DESCRIPTION OF THE DRAWINGS

The present invention will become more fully understood from the detailed description and the accompanying drawings.
Figure 1 shows SITA foam profiles of three toothpaste compositions (Composition I, Comparative composition I, and Comparative composition II) in DI-water.
Figure 2 shows SITA foam profiles of three toothpaste compositions (Composition I, Comparative composition I, and Comparative composition II) in artificial saliva.

### DETAILED DESCRIPTION

As used throughout, ranges are used as shorthand for describing each and every value that is within the range. Any value within the range can be selected as the terminus of the range.

Unless otherwise specified, all percentages and amounts expressed herein and elsewhere in the specification should be understood to refer to percentages by weight. The amounts given are based on the active weight of the material.

The present invention relates to viscosity stable toothpastes containing a zinc ion source and a basic amino acid as further defined in the claims. Zinc ions tend to react with ionic thickeners or binders like cellulose (CMC) or xanthan gum, leading to a loss of viscosity over time. It has been surprisingly found that the surfactant system of the invention stabilizes the viscosity of toothpastes over time. Without intending to be bound to theory, it is believed that SLS is a main driver for the unstable rheological condition of the toothpastes containing a zinc ion source, a basic amino acid and sodium lauryl sulfate. It is thus believed that the substitution of sodium lauryl sulfate by alternative surfactant systems which comprise (i) an alkyl glucoside and an acyl glutamate or (ii) a glycolipid, stabilizes the viscosity of toothpastes containing a zinc ion source and a basic amino acid.

Reaching a consumer accepted level of foaming is one of the key factors to consider during the development of SLS free toothpastes, especially in the case of zinc containing toothpastes, because foam formation is even additionally impaired by zinc ions. It has been found that the SLS free toothpastes of the invention yield a consumer accepted level of foaming. Thus, the surfactant system of the invention is a better alternative to sodium lauryl sulfate in an oral are composition containing a zinc ion source and a basic amino acid in order to stabilize the viscosity of the composition and improve the foaming properties of the composition.

The present invention provides, in an aspect, an oral care composition (Compositions 1.0), e.g., toothpaste or gel, comprising a basic amino acid in free or salt form, a zinc ion source and a surfactant system comprising one or more of alkyl glucosides e.g., lauryl glucoside, acyl glutamates, e.g., sodium cocoyl glutamate, glycolipids, e.g., rhamnolipid, or a combination thereof as further defined in the claims.

For example, the invention includes:
The surfactant system comprises an alkyl glucoside and an acyl glutamate or the surfactant system comprises a glycolipid as further defined in the claims.
The alkyl glucoside is lauryl glucoside as defined in the claims.
The alkyl glucoside, if present, is present in an amount of from 2% to 3%, e.g., from 2.2% to 2.8%, from 2.4% to 2.6%, or about 2.5% by weight of the composition as further defined in the claims.
The acyl glutamate is sodium cocoyl glutamate as further defined in the claims.
The acyl glutamate, if present, is present in an amount of from 2% to 3%, e.g., from 2.2% to 2.8%, from 2.4% to 2.6%, or about 2.5% by weight of the composition as further defined in the claims.
The glycolipid can be rhamnolipid, sophorolipid, trehalolipid, cellobiose lipid, or mannosylerythritol lipid.
The glycolipid can be rhamnolipid, e.g., potassium rhamnolipid.
The glycolipid, if present, is present in an amount of from 3% to 7%, from 4% to 6%, from 4% to 5%, or about 4.5% by weight of the composition as further defined in the claims.
The surfactant system comprises lauryl glucoside and sodium cocoyl glutamate as further defined in the claims.
The surfactant system can comprise a glycolipid, optionally wherein the surfactant system comprises rhamnolipid, e.g., potassium rhamnolipid.
The surfactant system can further comprise a betaine zwitterionic surfactant and a non-ionic block copolymer.
The betaine zwitterionic surfactant can be a C₈-C₁₆ amidopropyl betaine, optionally wherein the zwitterionic surfactant is cocamidopropyl betaine.
The betaine zwitterionic surfactant can be present in an amount of from 1% to 1.5%, from 1.1% to 1.4%, from 1.2% to 1.3%, or about 1.25% by weight of the composition. The non-ionic block copolymer can be a poly(propylene oxide)/poly(ethylene oxide) copolymer.
The non-ionic block copolymer can be a poloxamer.
The poloxamer can have a polyoxylpropylene molecular mass of from 3000 to 5000g/mol and a polyoxyethylene content of from 60 to 80 mol%, optionally wherein the poloxamer is poloxamer 407.
The non-ionic block copolymer can be present in an amount of from 0.3% to 0.7%, from 0.4% to 0.6%, or about 0.5% by weight of the composition.
The basic amino acid can further comprise one or more of lysine, citrulline, ornithine, creatine, histidine, diaminobutanoic acid, diaminopropionic acid, salts thereof, or combinations thereof.
The basic amino acid can have the L-configuration.
The basic amino acid is present in an amount of from 1% to 15% as further defined in the claims, e.g., from 1% to 10%, from 1% to 5%, from 1% to 3%, from 1% to 2%, from 1.2% to 1.8%, from 1.4% to 1.6%, or about 1.5% by weight of the composition, being calculated as free base form.
The basic amino acid comprises arginine as further defined in the claims.
The basic amino acid can comprise L-arginine.
The basic amino acid can comprise arginine bicarbonate, arginine phosphate, arginine sulfate, arginine hydrochloride or combinations thereof, optionally wherein the basic amino acid is arginine bicarbonate.
The zinc ion source can be selected from the group consisting of zinc oxide, zinc sulfate, zinc chloride, zinc citrate, zinc lactate, zinc gluconate, zinc malate, zinc tartrate, zinc carbonate, zinc phosphate and a combination thereof.
The zinc ion source can be present an amount of from 0.01 % to 5 %, e.g., 0.1% to 4%, or 0.5% to 3%, by weight of the composition.
The zinc ion source can be selected from the group consisting of zinc oxide, zinc citrate, and a combination thereof, optionally wherein the zinc ion source is a combination of zinc oxide and zinc citrate.
Zinc oxide can be present in an amount of 0.5 % to 2%, e.g., 0.5% to 1.5%, or about 1% by weight of the composition.
Zinc citrate can be present in an amount of 0.1% to 1%, 0.25 to 0.75%, or about 0.5% by weight of the composition.
The composition can comprise a fluoride ion source selected from sodium fluoride, stannous fluoride, potassium fluoride, sodium monofluorophosphate, sodium fluorosilicate, ammonium fluorosilicate, amine fluoride (e.g., N'-octadecyltrimethylendiamine-N,N,N'-tris(2-ethanol)-dihydrofluoride), ammonium fluoride, titanium fluoride, hexafluorosulfate, and a combination thereof.
The fluoride ion source can be present in an amount sufficient to supply 25 ppm to 5,000 ppm of fluoride ions, generally at least 500 ppm, e.g., 500 to 2000 ppm, e.g., 1000 ppm to 1600 ppm, e.g., 1450 ppm.
The fluoride ion source can be sodium fluoride.
The composition can comprise a thickener.
The thickener can comprise xanthan gum, optionally wherein xanthan gum is present in an amount of from 0.1 to 1%, from 0.2-0.8%, from 0.3% to 0.6%, from 0.3% to 0.5%, or about 0.4% by weight of the composition.
The thickener can comprise carboxymethyl cellulose, optionally wherein carboxymethyl cellulose is present in an amount of from 0.5% to 2%, from 0.8% to 1.5%, from 1% to 1.3%, from 1% to 1.2% or about 1.1% by weight of the composition.
The thickener can comprise xanthan gum in an amount of from 0.1 to 1%, from 0.2-0.8%, from 0.3% to 0.6%, from 0.3% to 0.5%, or about 0.4% by weight of the composition and carboxymethyl cellulose in an amount of from 0.5% to 2%, from 0.8% to 1.5%, from 1% to 1.3%, from 1% to 1.2% or about 1.1% by weight of the composition.
The thickener can comprise xanthan gum present in an amount of from 0.3% to 0.5% by weight of the composition and carboxymethyl cellulose in in an amount of from 1% to 1.2% by weight of the composition.
The thickener can further comprise a thickening silica, optionally wherein the thickening silica is present in an amount of from 5 to 10%, from 6% to 8% or about 7%, by weight of the composition, further optionally wherein the thickening silica is present in an amount of from 6% to 8% by weight of the composition.
The composition can comprise an abrasive.
The abrasive can be selected from silica abrasives, calcium phosphate abrasives, e.g., tricalcium phosphate (Ca₃(PO₄)₂), hydroxyapatite (Ca₁₀(PO₄)₆(OH)₂), or dicalcium phosphate dihydrate (CaHPO₄ • 2H₂O, also sometimes referred to herein as DiCal) or calcium pyrophosphate; calcium carbonate abrasive; or abrasives such as sodium metaphosphate, potassium metaphosphate, aluminum silicate, calcined alumina, bentonite or other siliceous materials, and combinations thereof.
The abrasive can be present in an amount of from 10% to 70%, e.g., from 10 % to 30 %, e.g., 10% to 20%, 15% to 25%, from 20% to 50%, from 25% to 45%, or from 30% to 40% by weight of the composition.
The abrasive can comprise a silica abrasive.
The abrasive can be a calcium-free silica abrasive.
The silica abrasive can be present in an amount of from 10 % to 30 %, e.g., 10% to 20%, 15% to 25%, or about 15%, by weight of the composition.
The composition can be substantially free of calcium, e.g., comprises less than 2%, less than 1%, less than 0.5%, or less than 0.1% calcium by weight of the composition.
The composition can comprise a humectant, optionally wherein the humectant is selected from sorbitol, glycerin and a mixture thereof.
The humectant can comprise glycerin, optionally wherein glycerin is present in an amount of from 15% to 40%, from 20% to 40%, from 30% to 40%, or about 35% by weight of the composition.
The composition can comprise one or more soluble phosphate salts, e.g. selected from tetrasodium pyrophosphate (TSPP), sodium tripolyphosphate (STPP) and a combination thereof.
The composition can comprise water, optionally wherein water is present in an amount of from 10% to 80%, from 20% to 60%, from 20% to 40%, from 10% to 30%, from 20% to 30% or from 25% to 35% by weight of the composition.
The composition is free of sodium lauryl sulfate as defined in the claims.
The composition can be free or substantially free of alkyl sulfate salts.
The composition can be a toothpaste or gel.
The composition can be a toothpaste.
The compositions can be for use in (i) reducing or inhibiting formation of dental caries, (ii) reducing, repairing or inhibiting pre-carious lesions of the enamel, (iii) reducing or inhibiting demineralization and promote remineralization of the teeth, (iv) reducing hypersensitivity of the teeth, (v) reducing or inhibiting gingivitis, (vi) promoting healing of sores or cuts in the oral cavity, (vii) reducing levels of acid producing bacteria, (viii) reducing or inhibiting microbial biofilm formation in the oral cavity, (ix) reducing or inhibiting plaque formation in the oral cavity, (x) promoting systemic health, or (xi) cleaning teeth and oral cavity.

The oral care composition of the invention can be in the form of any oral care formulations, including dentifrice, toothpaste, gel, mouthwash, powder, cream, strip, gum, bead, film, floss or any other known in the art. In some embodiments, the oral care composition is a toothpaste or gel. In some embodiments, the oral care composition is a toothpaste.

The oral care composition of the invention may be a single phase oral care composition. For example, all the components of the oral care composition may be maintained together with one another in a single phase and/or vessel. For example, all the components of the oral care composition may be maintained in a single phase, such as a single homogenous phase. In another embodiment, the oral care composition may be a multi-phase oral care composition.

The oral care composition of the invention may contain an orally acceptable carrier. As used herein, an "orally acceptable carrier" refers to a material or combination of materials that are safe for use in the compositions of the invention, commensurate with a reasonable benefit/risk ratio. Such materials include but are not limited to, for example, water, humectants, ionic active ingredients, buffering agents, anticalculus agents, abrasive polishing materials, peroxide sources, alkali metal bicarbonate salts, surfactants, titanium dioxide, coloring agents, flavor systems, sweetening agents, antimicrobial agents, herbal agents, desensitizing agents, stain reducing agents, and mixtures thereof. Such materials are well known in the art and are readily chosen by one skilled in the art based on the physical and aesthetic properties desired for the compositions being prepared. In some embodiment, the orally acceptable carrier may include an orally acceptable solvent. Illustrative solvents may include, but are not limited to, one or more of ethanol, phenoxyethanol, isopropanol, water, cyclohexane, methyl glycol acetate, benzyl alcohol, or the like, or any mixture or combination thereof. In a particular embodiment, the orally acceptable solvent includes benzyl alcohol.

Water may be present in the oral compositions of the invention. Water employed in the preparation of commercial oral compositions should be deionized and free of organic impurities. Water commonly makes up the balance of the compositions and includes about 10% to about 80%, about 20% to about 60%, about 20% to 40%, about 10% to about 30%, about 20% to 30%, or about 25% to 35% by weight of the oral compositions. This amount of water includes the free water which is added plus that amount which is introduced with other materials such as with sorbitol or any components of the invention.

The oral care composition of the invention comprises a basic amino acid in free or salt form as further defined in the claims. The basic amino acids which can be used in the compositions include not only naturally occurring basic amino acids, such as arginine, lysine, and histidine, but also any basic amino acids having a carboxyl group and an amino group in the molecule, which are water-soluble and provide an aqueous solution with a pH of about 7 or greater. Accordingly, basic amino acids include, but are not limited to, arginine, lysine, citrulline, ornithine, creatine, histidine, diaminobutanoic acid, diaminopropionic acid, salts thereof or combinations thereof. In a particular embodiment, the basic amino acid as defined in the claims comprises arginine as defined in the claims and further basic amino acids can be selected from lysine, citrulline, and ornithine. The basic amino acids of the oral care composition may generally be present in the L-form or L-configuration. The basic amino acids may be provided as a salt of a di- or tri-peptide including the amino acid. In some embodiments, at least a portion of the basic amino acid present in the oral care composition is in a salt form. In some embodiments, the basic amino acid is arginine, for example, L-arginine, or a salt thereof. Arginine may be provided as free arginine or a salt thereof. For example, Arginine may be provided as arginine phosphate, arginine hydrochloride, arginine sulfate, arginine bicarbonate, or the like, and mixtures or combinations thereof. The basic amino acid may be provided as a solution or a solid. For example, the basic amino acid may be provided as an aqueous solution. In some embodiment, the amino acid includes or is provided by an arginine bicarbonate solution. For example, the amino acid may be provided by an about 40% solution of the basic amino acid, such as arginine bicarbonate or alternatively called as arginine carbamate. In some embodiments, the basic amino acid is present in an amount of from 1% to 15%, e.g., from 1% to 10%, from 1% to 5%, from 1% to 3%, from 1% to 2%, from 1.2% to 1.8%, from 1.4% to 1.6%, or about 1.5% by weight of the composition, being calculated as free base form.

The oral care composition of the invention comprises a zinc ion source. The zinc ion source may be or include a zinc ion and/or one or more zinc salts. For example, the zinc salts may at least partially dissociate in an aqueous solution to produce zinc ions. Illustrative zinc salts may include, but are not limited to, zinc lactate, zinc oxide, zinc chloride, zinc phosphate, zinc citrate, zinc acetate, zinc borate, zinc butyrate, zinc carbonate, zinc formate, zinc gluconate, zinc glycerate, zinc glycolate, zinc picolinate, zinc propionate, zinc salicylate, zinc silicate, zinc stearate, zinc tartrate, zinc undecylenate, and mixtures thereof. In some embodiments, the zinc ion source is present in an amount of from 0.01 % to 5 %, e.g., 0.1% to 4%, or 1% to 3%, by weight of the composition.

In some embodiments, the zinc ion source is selected from zinc oxide, zinc citrate, and a combination thereof. Zinc oxide may be present in an amount of 0.5 % to 2%, e.g., 0.5% to 1.5%, or about 1% by weight of the composition. Zinc citrate may be present in an amount of 0.1% to 1%, 0.25% to 0.75%, about 0.5% by weight of the composition by weight of the composition. In some embodiments, the composition comprises zinc oxide and zinc citrate. The composition may comprise zinc oxide in an amount of 0.5 % to 2%, e.g., 0.5% to 1.5%, about 1% or about 1.2% by weight of the composition and zinc citrate in an amount of 0.1% to 1%, 0.25% to 0.75%, about 0.5% by weight of the composition. In certain embodiments, the composition comprises zinc oxide in an amount of about 1% by weight of the composition and zinc citrate in an amount of about 0.5% by weight of the composition.

The oral care composition of the invention comprises a surfactant system comprising lauryl glucoside and sodium cocoyl glutamate; or a glycolipid as further defined in the claims. As used herein, the terms "acyl glutamate" and "glycolipid" may be or include free form or any orally acceptable salts thereof. Alkyl glucosides which can be used in the composition may be C₈₋₂₅ alkyl glucoside, e.g., C₈₋₁₈ alkyl glucoside, or C₁₀₋₁₈ alkyl glucoside. The alkyl glucoside may be present in an amount of from 2% to 3%, e.g., from 2.2% to 2.8%, from 2.4% to 2.6%, or about 2.5% by weight of the composition. In some embodiments, the alkyl glucoside is lauryl glucoside. Acyl glutamates which can be used in the compositions may be C₈₋₂₅ acyl glutamate, e.g., C₈₋₁₈ acyl glutamate, or C₁₀₋₁₈ acyl glutamate. The acyl glutamate may be present in an amount of from 2% to 3%, e.g., from 2.2% to 2.8%, from 2.4% to 2.6%, or about 2.5% by weight of the composition. In some embodiments, the acyl glutamate is cocoyl glutamate, e.g., sodium cocoyl glutamate. The term "glycolipid" refers to any of a class of lipids that, upon hydrolysis, yield a sugar (e.g., galactose or glucose), and a lipid. The glycolipid may be present in an amount of from 3% to 7%, from 4% to 6%, from 4% to 5%, or about 4.5% by weight of the composition. In some embodiments, the glycolipid may be rhamnolipid, sophorolipid, trehalolipid, cellobiose lipid, or mannosylerythritol lipid. In some embodiments, the glycolipid is rhamnolipid, e.g., potassium rhamnolipid.

The surfactant system comprises an alkyl glucoside and an acyl glutamate or the surfactant system comprises a glycolipid as further defined in the claims. In some embodiments, the surfactant system comprises a glucoside and an acyl glutamate as further defined in the claims. In some embodiments, the surfactant system comprises lauryl glucoside, in an amount of from 2% to 3%, e.g., from 2.2% to 2.8%, from 2.4% to 2.6%, or about 2.5% by weight of the composition and sodium cocoyl glutamate, in an amount of from 2% to 3%, e.g., from 2.2% to 2.8%, from 2.4% to 2.6%, or about 2.5% by weight of the composition. In some embodiments, the surfactant system comprises lauryl glucoside and cocoyl glutamate, e.g., sodium cocoyl glutamate as further defined in the claims. In some embodiments, the surfactant system comprises a glycolipid as further defined in the claims. In some embodiments, the surfactant system comprises a glycolipid in an amount of from 3% to 7%, from 4% to 6%, from 4% to 5%, or about 4.5% by weight of the composition. In some embodiments, the glycolipid is rhamnolipid, e.g., potassium rhamnolipid.

Additional surfactants, which may be zwitterionic or nonionic, and are known for use in oral care compositions, may be included in the oral care composition of the invention. In some embodiments, the surfactant system further comprises a betaine zwitterionic surfactant and a non-ionic block copolymer. The betaine zwitterionic surfactant may be a C₈-C₁₆ aminopropyl betaine, e.g., cocamidopropyl betaine. In some embodiments, the betaine zwitterionic surfactant, e.g., cocamidopropyl betaine, is present in an amount of from 1% to 1.5%, from 1.1% to 1.4%, from 1.2% to 1.3%, or about 1.25% by weight of the composition. The non-ionic block copolymer may be a poly(propylene oxide)/poly(ethylene oxide) copolymer. In some embodiments, the copolymer has a polyoxypropylene molecular mass of from 3000 to 5000 g/mol and a polyoxyethylene content of from 60 to 80 mol%. In some embodiments, the non-ionic block copolymer is a poloxamer. In some embodiments, the non-ionic block copolymer is selected from: Poloxamer 338, Poloxamer 407, Poloxamer, 237, Poloxamer, 217, Poloxamer 124, Poloxamer 184, Poloxamer 185, and a combination of two or more thereof. In some embodiments, the copolymer is Poloxamer 407, which is available in commerce as Pluronic F-127 (Pluronic is a trademark of BASF Corporation). In some embodiments, the non-ionic block copolymer, e.g., poloxamer, e.g., poloxamer 407, is present in an amount of from 0.3% to 0.7%, from 0.4% to 0.6%, or about 0.5% by weight of the composition.

The oral care composition of the invention is free of sodium lauryl sulfate as further defined in the claims. In some embodiments, the oral care composition may be free or substantially free of alkyl sulfate salts. As used herein, "substantially free" of a material may refer to a composition where the material is present in an amount of less than 0.1 weight %, less than 0.05 weight %, less than 0.01 weight %, less than 0.005 weight %, less than 0.001 weight %, or less than 0.0001 weight % based on a total weight of the composition.

The oral care composition of the invention may be free or substantially free of fluoride (e.g., soluble fluoride salts). In another embodiment, the oral care composition may include fluoride, such as one or more fluoride ion sources (e.g., soluble fluoride salts). A wide variety of fluoride ion-yielding materials may be employed as sources of soluble fluoride. Illustrative fluoride ion sources include, but are not limited to, sodium fluoride, stannous fluoride, potassium fluoride, sodium monofluorophosphate, fluorosilicate salts, such as sodium fluorosilicate and ammonium fluorosilicate, amine fluoride, ammonium fluoride, and combinations thereof. In some embodiment, the fluoride ion source includes sodium fluoride. The amount of the fluoride ion source present in the oral care composition may be greater than 0 weight % and less than 0.8 wt.%, less than 0.7 wt.%, less than 0.6 wt.%, less than 0.5 wt.%, or less than 0.4 wt.%. The fluoride ion sources may be present in an amount sufficient to supply 25 ppm to 5,000 ppm of fluoride ions, generally at least 500 ppm, e.g., 500 to 2000 ppm, e.g., 1000 ppm to 1600 ppm, e.g., 1450 ppm.

The oral care composition of the invention may include thickeners. Suitable thickeners may be any orally acceptable thickener or thickening agent configured to control the viscosity of the oral care composition. Illustrative thickeners may be or include, but are not limited to, colloidal silica, fumed silica, a cross-linked polyvinylpyrrolidone (PVP) polymer, cross-linked polyvinylpyrrolidone (PVP), or the like, or mixtures or combinations thereof. In some embodiments, the thickening system includes a cross-linked polyvinylpyrrolidone (PVP) polymer. The thickening system may also include POLYPLASDONE^{®} XL 10F, which is commercially available from Ashland Inc. of Covington, KY. Illustrative thickeners may also be or include, but are not limited to, carbomers (*e.g.,* carboxyvinyl polymers), carrageenans (*e.g.,* Irish moss, carrageenan, iota-carrageenan, *etc*.), high molecular weight polyethylene glycols (*e.g.,* CARBOWAX^{®}, which is commercially available from The Dow Chemical Company of Midland, MI), cellulosic polymers, hydroxyethylcellulose, carboxymethylcellulose, and salts thereof *(e.g.,* CMC sodium), natural gums *(e.g.,* karaya, xanthan, gum arabic, and tragacanth), colloidal magnesium aluminum silicate, or the like, or mixtures or combinations thereof. Thickeners particularly suitable of use in the oral care composition of the invention include natural and synthetic gums and colloids. Optionally, the composition comprises at least one gum selected from carrageenan and xanthan gum.

In some embodiments, the composition comprises xanthan gum. Xanthan gum may be present in an amount of from 0.1 to 1%, from 0.2-0.8%, from 0.3% to 0.6%, from 0.3% to 0.5%, or about 0.4% by weight of the composition. In some embodiments, the composition comprises carboxymethyl cellulose. Carboxymethyl cellulose may be present in an amount of from 0.5% to 2%, from 0.8% to 1.5%, from 1% to 1.3%, from 1% to 1.2% or about 1.1% by weight of the composition. In some embodiments, the composition comprises xanthan gum in an amount of from 0.1 to 1%, from 0.2-0.8%, from 0.3% to 0.6%, from 0.3% to 0.5%, or about 0.4% by weight of the composition and carboxymethyl cellulose in an amount of from 0.5% to 2%, from 0.8% to 1.5%, from 1% to 1.3%, from 1% to 1.2% or about 1.1% by weight of the composition. In certain embodiments, the composition comprises xanthan in an amount of from 0.3% to 0.5% by weight of the composition and carboxymethyl cellulose in in an amount of from 1% to 1.2% by weight of the composition. In some embodiments, the composition comprises a thickening silica, optionally wherein the thickening silica is present in an amount of from 5 to 10%, from 6% to 8% or about 7%, by weight of the composition. In some embodiments, the composition comprises xanthan gum present in an amount of from 0.1 to 1%, from 0.2-0.8%, from 0.3% to 0.6%, from 0.3% to 0.5%, or about 0.4% by weight of the composition, carboxymethyl cellulose in in an amount of from 0.5% to 2%, from 0.8% to 1.5%, from 1% to 1.3%, from 1% to 1.2% or about 1.1% by weight of the composition, and a thickening silica in an amount of from 5 to 10%, from 6% to 8% or about 7%, by weight of the composition. In certain embodiments, the composition comprises xanthan gum present in an amount of from 0.3% to 0.5% by weight of the composition, carboxymethyl cellulose in in an amount of from 1% to 1.2% by weight of the composition, and a thickening silica in an amount of from 6% to 8% by weight of the composition.

In some embodiments, the oral care compositions may include one or more abrasives or an abrasive system including one or more abrasives. As used herein, the term "abrasive" may also refer to materials commonly referred to as "polishing agents". Any orally acceptable abrasive may be used, but preferably, type, fineness (particle size), and amount of the abrasive may be selected such that the tooth enamel is not excessively abraded in normal use of the oral care composition.
The one or more abrasives may have a particle size or D50 of less than or equal to about 10 µm, less than or equal to about 8 µm, less than or equal to about 5 µm, or less than or equal to about 3 µm. The one or more abrasives may have a particle size or D50 of greater than or equal to about 0.01 µm, greater than or equal to about 0.05 µm, greater than or equal to about 0.1 µm, greater than or equal to about 0.5 µm, or greater than or equal to about 1 µm. Illustrative abrasives may include, but are not limited to, metaphosphate compounds, phosphate salts (e.g., insoluble phosphate salts), such as sodium metaphosphate, potassium metaphosphate, calcium pyrophosphate, magnesium orthophosphate, trimagnesium orthophosphate, tricalcium phosphate, dicalcium phosphate dihydrate, anhydrous dicalcium phosphate, calcium carbonate (e.g., precipitated calcium carbonate and/or natural calcium carbonate), magnesium carbonate, hydrated alumina, silica, zirconium silicate, aluminum silicate including calcined aluminum silicate, polymethyl methacrylate, or the like, or mixtures and combinations thereof. In some embodiments, the oral care composition comprises a silica abrasive. In some embodiments, the silica abrasive is present in an amount of from 10 % to 30 %, e.g., 10% to 20%, 15% to 25%, or about 15%, by weight of the composition. In some embodiments, the oral care composition comprises a calcium-free silica abrasive. In some embodiments, the composition is substantially free of calcium, e.g., comprises less than 2%, less than 1%, less than 0.5%, or less than 0.1% calcium by weight of the composition.

In some embodiments, the oral care composition of the invention comprises a calcium-containing abrasive (e.g., calcium carbonate). In some embodiments, the calcium-containing abrasive is selected from calcium carbonate, calcium phosphate (e.g., dicalcium phosphate dihydrate), calcium sulfate, and combinations thereof. In some embodiments, the oral care composition comprises calcium carbonate as an abrasive. In one embodiment, the oral care composition comprises precipitated calcium carbonate or natural calcium carbonate. Precipitated calcium carbonate may be preferred over natural calcium carbonate.

The amount or concentration of the one or more abrasives present in the oral care composition may vary widely. In some embodiments, the amount of the abrasives present in the oral care composition may be from about 15 weight % to about 70 weight %, e.g., from about 20 weight % to about 50weight %, from about 25 weight % to about 45 weight %, from about 30 weight % to about 40 weight %, from about 10% to about 20 weight%, or about 15 weight %, based on a total weight of the oral care composition.

In some embodiments, the oral care compositions of the invention may include one or more humectants. Humectants can reduce evaporation and also contribute towards preservation by lowering water activity and can also impart desirable sweetness or flavor to compositions. Illustrative humectants may be or include, but are not limited to, glycerin, propylene glycol, polyethylene glycol, sorbitol, xylitol, or the like, or any mixture or combination thereof. In a preferred embodiment, the orally acceptable vehicle may be or include, but is not limited to, glycerin or sorbitol. In some embodiments, the humectant is selected from glycerin, sorbitol and a combination thereof. In some embodiments, the humectant may be present in an amount of from 20% to 60%, for example from 15% to 40%, from 15% to 35%, from 20% to 40%, from 30% to 50%, from 30% to 40%, or from 40% to 45%, by weight of the composition. In some embodiments, the composition comprises glycerin, optionally wherein glycerin is present in an amount of from 15% to 40%, from 20% to 40%, from 30% to 40%, or about 35% by weight of the composition.

The oral care compositions of the present invention may include a preservative. Suitable preservatives include, but are not limited to, sodium benzoate, potassium sorbate, methylisothiazolinone, paraben preservatives, for example methyl p-hydroxybenzoate, propyl p-hydroxybenzoate, and mixtures thereof.

The oral care compositions of the present invention may include a sweetener such as, for example, saccharin, for example sodium saccharin, acesulfame, neotame, cyclamate or sucralose; natural high-intensity sweeteners such as thaumatin, stevioside or glycyrrhizin; or such as sorbitol, xylitol, maltitol or mannitol. One or more of such sweeteners may be present in an amount of from 0.005% to 5% by weight, for example 0.01% to 1%, for example 0.01% to 0.5%, by weight of the composition.

The oral care compositions of the present invention may include a flavoring agent. Suitable flavoring agents include, but are not limited to, essential oils and various flavoring aldehydes, esters, alcohols, and similar materials, as well as sweeteners such as sodium saccharin. Examples of the essential oils include oils of spearmint, peppermint, wintergreen, sassafras, clove, sage, eucalyptus, marjoram, cinnamon, lemon, lime, grapefruit, and orange. Also useful are such chemicals as menthol, carvone, and anethole. The flavoring agent is typically incorporated in the oral composition at a concentration of 0.01 to 3% by weight.

The oral care composition of the invention may include one or more pH modifying agents. For example, the oral care composition may include one or more acidifying agents and/or one or more basifying agents configured to reduce and/or increase the pH thereof, respectively. Illustrative acidifying agents and/or one or more basifying agents may be or include, but are not limited to, an alkali metal hydroxide, such as sodium hydroxide and/or potassium hydroxide, citric acid, hydrochloric acid, or the like, or combinations thereof.

The oral care composition of the invention may also include one or more buffering agents configured to control or modulate the pH within a predetermined or desired range. Illustrative buffering agents may include, but are not limited to, sodium bicarbonate, sodium phosphate, sodium carbonate, sodium acid pyrophosphate, sodium citrate, and mixtures thereof. Sodium phosphate may include monosodium phosphate (NaH₂PO₄), disodium phosphate (Na₂HPO₄), trisodium phosphate (Na₃PO₄), and mixtures thereof. In a typical embodiment, the buffering agent may be anhydrous sodium phosphate dibasic or disodium phosphate and/or sodium phosphate monobasic. In another embodiment, the buffering agent includes anhydrous sodium phosphate dibasic or disodium phosphate, and phosphoric acid (e.g., syrupy phosphoric acid; 85%-Food Grade).

The oral care composition of the invention may include anticalculus agents. Illustrative anticalculus agents may include, but are not limited to, phosphates and polyphosphates (e.g., pyrophosphates), polyaminopropanesulfonic acid (AMPS), hexametaphosphate salts, zinc citrate trihydrate, polypeptides, polyolefin sulfonates, polyolefin phosphates, diphosphonates. In some embodiments, the anticalculus agent includes tetrasodium pyrophosphate (TSPP), sodium tripolyphosphate (STPP), or a combination thereof.

The oral care composition of the invention may include an antioxidant. Any orally acceptable antioxidant may be used, including, but not limited to, butylated hydroxyanisole (BHA), butylated hydroxytoluene (BHT), vitamin A, carotenoids, vitamin E, flavonoids, polyphenols, ascorbic acid, herbal antioxidants, chlorophyll, melatonin, or the like, or combinations and mixtures thereof.

The oral care composition of the invention may include one or more pigments, such as whitening pigments. In some embodiments, the whitening pigments include particles ranging in size from about 0.1 µm to about 10 µm with a refractive index greater than about 1.2. Suitable whitening agents include, without limitation, titanium dioxide particles, zinc oxide particles, aluminum oxide particles, tin oxide particles, calcium oxide particles, magnesium oxide particles, barium oxide particles, silica particles, zirconium silicate particles, mica particles, talc particles, tetracalcium phosphate particles, amorphous calcium phosphate particles, alpha-tricalcium phosphate particles, beta-tricalcium phosphate particles, hydroxyapatite particles, calcium carbonate particles, zinc phosphate particles, silicon dioxide particles, zirconium silicate particles, or the like, or mixtures and combinations thereof. The whitening pigment, such as titanium dioxide particles, may be present in an amount that is sufficient to whiten the teeth.

All ingredients for use in the compositions described herein should be orally acceptable. As used herein, "orally acceptable" may refer any ingredient that is present in a composition as described in an amount and form which does not render the composition unsafe for use in the oral cavity.

The composition can be used in a method to (i) reduce or inhibit formation of dental caries, (ii) reduce, repair or inhibit pre-carious lesions of the enamel, (iii) reduce or inhibit demineralization and promote remineralization of the teeth, (iv) reduce hypersensitivity of the teeth, (v) reduce or inhibit gingivitis, (vi) promote healing of sores or cuts in the oral cavity, (vii) reduce levels of acid producing bacteria, (viii) reduce or inhibit microbial biofilm formation in the oral cavity, (ix) reduce or inhibit plaque formation in the oral cavity, (x) promote systemic health, or (xi) clean teeth and oral cavity, comprising applying an effective amount of any of oral care compositions as disclosed herein to the oral cavity of a subject in need thereof. The method may include contacting the oral care composition with water. The method may also include contacting the surface of the teeth with the oral care composition. Contacting the surface of the teeth with the oral care composition may include disposing the oral care composition (e.g., toothpaste) on a toothbrush and brushing the teeth with the toothbrush. The oral care composition may be applied and/or contacted with the surfaces of the teeth at predetermined intervals. For example, a daily basis, at least once a day, twice a day, or more, for multiple days, or alternatively every other day. In another example, the oral care composition may be applied and/or contacted with the surfaces of the teeth at least once a day, at least once every two days, at least once every three days, at least once every five days, at least once a week, at least once every two weeks, or at least once a month. The oral care composition thereof may be utilized for up to 2 weeks, up to 3 weeks, up to 4 weeks, up to 6 weeks, up to 8 weeks, or greater.

The oral care compositions as disclosed herein can be used to (i) reduce or inhibit formation of dental caries, (ii) reduce, repair or inhibit pre-carious lesions of the enamel, (iii) reduce or inhibit demineralization and promote remineralization of the teeth, (iv) reduce hypersensitivity of the teeth, (v) reduce or inhibit gingivitis, (vi) promote healing of sores or cuts in the oral cavity, (vii) reduce levels of acid producing bacteria, (viii) reduce or inhibit microbial biofilm formation in the oral cavity, (ix) reduce or inhibit plaque formation in the oral cavity, (x) promote systemic health, or (xi) clean teeth and oral cavity, in a subject in need thereof.

A surfactant system comprising one or more of alkyl glucosides e.g., lauryl glucoside, acyl glutamates, e.g., sodium cocoyl glutamate, glycolipids, e.g., rhamnolipid, or a combination thereof, e.g., any of surfactant systems as disclosed herein, can be used in the manufacture of an oral care composition, e.g., toothpaste or gel, comprising a basic amino acid in free or salt form and a zinc ion source, e.g., any of oral care compositions as disclosed herein.

A surfactant system comprising one or more of alkyl glucosides e.g., lauryl glucoside, acyl glutamates, e.g., sodium cocoyl glutamate, glycolipids, e.g., rhamnolipid, or a combination thereof, e.g., any of surfactant systems as disclosed herein, can be used in an oral care composition, e.g., toothpaste or gel comprising a basic amino acid in free or salt form and a zinc ion source, e.g., any of oral care compositions as disclosed herein, for stabilizing the viscosity of the composition and increasing the foaming of the composition in an oral cavity when applying the composition to the oral cavity.

### EXAMPLES

### Example 1

Two exemplary silica-based toothpastes of the invention having the formulations as indicated in Table 1 were prepared.

**Table 1**

| ingredient | Composition I (wt.%) | Composition II (wt.%) |
|---|---|---|
| Water | 27.61 | 28.11 |
| Glycerin | 35 | 35 |
| Xanthan gum | 0.4 | 0.4 |
| Sodium CMC-type 12 | 1.1 | 1.1 |
| Thickening silica | 7 | 7 |
| Abrasive silica | 15 | 15 |
| Tetrasodium pyrophosphate | 0.5 | 0.5 |
| Sodium fluoride | 0.32 | 0.32 |
| L-arginine | 1.5 | 1.5 |
| zinc oxide | 1 | 1 |
| Zinc citrate trihydrate | 0.5 | 0.5 |
| Poloxamer 407 | 0.5 | 0.5 |
| Cocamidopropyl betaine | 1.25 | 1.25 |
| Lauryl glucoside | 2.5 | 0 |
| Sodium cocoyl glutamate | 2.5 | 0 |
| Aqueous Rhamnolipid solution | 0 | 4.5 |
| Flavor, sweetener, preservative and colors | Balance | Balance |

Compositions I and II contain 15% abrasive silica, 1.5% L-arginine, 1% zinc oxide and 0.5% zinc citrate. These compositions contain 1.25% cocamidopropyl betaine and 0.5% poloxamer 407 but do not contain SLS. Instead, Composition I contains 2.5% lauryl glucoside and 2.5% sodium cocoyl glutamate as alternative surfactants, and Composition II contains 4.5% rhamnolipid (Rheance One, Evonik) as an alternative surfactant. Both toothpastes passed a flavor test based on 6 week aging at 49 °C, showing that the toothpastes will not alienate the consumers.

The viscosities of the two exemplary toothpastes of the invention and a SLS-containing comparative toothpaste (Comparative composition I) were compared. Comparative Composition I contains 2% SLS instead of the above mentioned alternative surfactants (lauryl glucoside, sodium cocoyl glutamate or rhamnolipid) but is otherwise identical to Compositions I and II. The viscosities of the toothpastes were monitored at room temperature, 40 °C, or 49 °C over time up to 90 days as indicated in Tables 2 and 3. Viscosity was measured at room temperature by Brookfield viscometer using the V74 spindle flow method and the results are shown in Tables 2 and 3.

**Table 2. Viscosity monitored at RT and 40 °C over time**

| Fit Flow Viscosity cP | | | | | | |
|---|---|---|---|---|---|---|
| | Composition I | | Composition II | | Comparative composition | |
| Condition | RT | 40°C | RT | 40°C | RT | 40°C |
| Day 0 | 422440 | | 356615 | | 402198 | |
| Day 14 | 364612 | | 360911 | | | |
| Day 30 | 399771 | 364414 | 518996 | 483506 | | 278895 |
| Day 60 | 364216 | 377632 | 454757 | 444249 | | 264620 |
| Day 90 | 361836 | 349742 | 436517 | 415501 | 269114 | 267726 |
| Viscosity loss at 40°C | | 17% | | -17% | | 33% |

**Table 3 Viscosity drop stress test with 4 weeks aging at 49 °C**

| | Composition I | | Composition II | | Comparative composition I | |
|---|---|---|---|---|---|---|
| Condition | Initial | 4 weeks 49°C | Initial | 4 weeks 49°C | Initial | 4 weeks 49°C |
| Fit Flow Viscosity cP | 422440 | 359589 | 356615 | 526728 | 414094 | 266244 |
| Viscosity loss at 49°C | | 15% | | -48% | | 36% |

As shown in Tables 2 and 3, Comparative composition I (2% SLS) lost its viscosity by 33% or 36% over time, while Compositions I and II either showed a much smaller viscosity loss (Composition I) or even built its viscosity. The results show that the substitution of SLS by the alternative surfactants (lauryl glucoside and sodium cocoyl glutamate (Composition I) or rhamnolipid (Composition II)) stabilized the viscosity, suggesting that SLS is an essential driver for the unstable rheological condition of toothpastes containing zinc compounds and arginine. For all tested compositions, the viscosity loss after 4 weeks at 49 °C was close to the viscosity loss after 90 days at 40 °C, indicating that the toothpastes have reached equilibrium and a further drop is not expected. This is an important advantage as for state of the art product, additional thickeners have to be added to compensate for the later occurring viscosity loss. The surfactant system of the invention makes this additional viscosity buffer dispensable. This also avoids issues during manufacturing as a less thick toothpaste that is easier to pump and fill will be obtained.

### Example 2

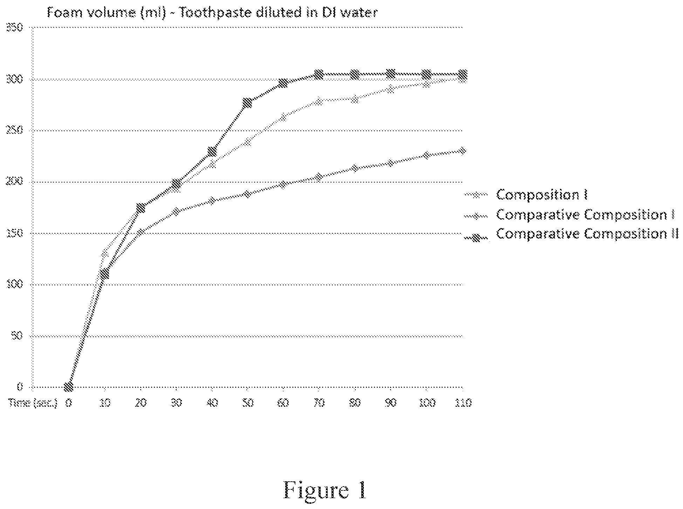

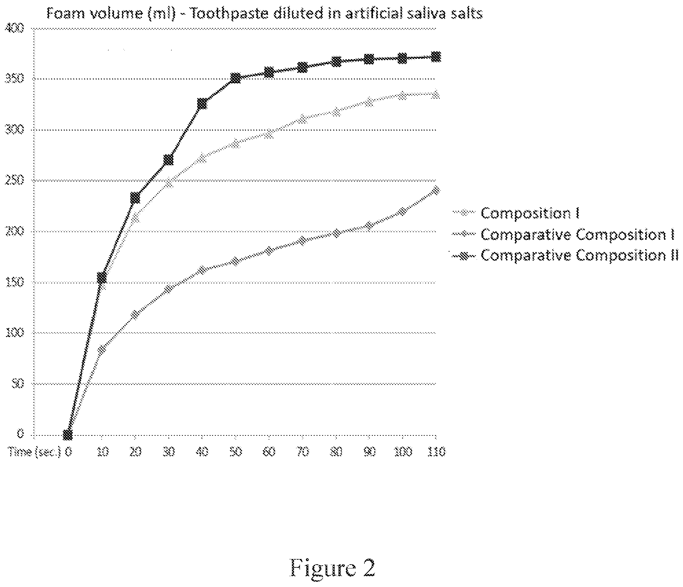

A SITA foam building and volume test was performed with Composition I (2.5% lauryl glucoside and 2.5% sodium cocoyl glutamate), Comparative composition I (2% SLS), and a marketed toothpaste (Colgate Total) containing 1.5 % SLS and no zinc (Comparative composition II). The test was performed both in deionized water and artificial saliva to check for robustness under clinical conditions. For each test, foam volume was measured at various time points. The results are shown in Figures 1 and 2. As can be seen from Figures 1 and 2, deionized water and artificial saliva yielded comparable results. In both conditions, Composition 1 (2.5% lauryl glucoside and 2.5% sodium cocoyl glutamate) generated more foam at a higher rate than Comparative composition II (1.5% SLS, no zinc) and almost reached the foam profile of Comparative composition (2.0% SLS). This result shows that the SLS free toothpaste of the invention will not suffer from consumer alienation due to an altered foam level.

## Claims

1. An oral care composition comprising a basic amino acid in free or salt form, wherein the basic amino acid comprises arginine and is present in an amount from 1% to 15% by weight of the composition, being calculated as free base form;
a zinc ion source and
a surfactant system comprising lauryl glucoside and sodium cocoyl glutamate; or a glycolipid;
wherein lauryl glucoside, if present, is present in an amount of from 2% to 3% by weight of the composition and sodium cocoyl glutamate, if present, is present in an amount of from 2% to 3% by weight of the composition;
wherein the glycolipid, if present, is present in an amount of from 3% to 7% by weight of the composition; and
wherein the composition is free of sodium lauryl sulfate.

2. The composition of claim 1, wherein the glycolipid is rhamnolipid.

3. The composition of any one of the preceding claims, wherein the surfactant system further comprises a betaine zwitterionic surfactant and a non-ionic block copolymer.

4. The composition of claim 3, wherein the betaine zwitterionic surfactant is present in an amount of from 1% to 1.5% by weight of the composition and the non-ionic block copolymer is present in an amount of from 0.3% to 0.7% by weight of the composition.

5. The composition of claim 3 or claim 4, wherein the betaine zwitterionic surfactant is cocamidopropyl betaine and the non-ionic block copolymer is a poly(propylene oxide)/poly(ethylene oxide) copolymer.

6. The composition of any one of the preceding claims, wherein the zinc ion source is selected from the group consisting of zinc oxide, zinc sulfate, zinc chloride, zinc citrate, zinc lactate, zinc gluconate, zinc malate, zinc tartrate, zinc carbonate, zinc phosphate and a combination thereof.

7. The composition of claim 6, wherein the zinc ion source is selected from the group consisting of zinc oxide, zinc citrate, and a combination thereof.

8. The composition of claim 7, wherein the composition comprises zinc oxide in an amount of from 0.5 % to 2% by weight of the composition and zinc citrate in an amount of from 0.1% to 1% by weight of the composition.

9. The composition of any one of the preceding claims, wherein the composition comprises a fluoride ion source.

10. The composition of any one of the preceding claims, wherein the composition comprises a thickening agent, optionally wherein the thickening agent comprises xanthan gum and carboxymethyl cellulose.

11. The composition of any one of the preceding claims, wherein the composition comprises a silica abrasive.

12. The oral care composition of any one of the preceding claims, wherein the oral care composition is a toothpaste.

## Patentansprüche

1. Mundpflegezusammensetzung, umfassend eine basische Aminosäure in freier Form oder Salzform, wobei die basische Aminosäure Arginin umfasst und in einer Menge von 1 bis 15 Gew.-%, bezogen auf das Gewicht der Zusammensetzung, vorliegt, berechnet als freie Base;
eine Zinkionenquelle; und
ein Tensidsystem, umfassend Laurylglucosid und Natriumcocoylglutamat; oder ein Glykolipid;
wobei Laurylglucosid, sofern vorhanden, in einer Menge von 2 bis 3 Gew.-%, bezogen auf das Gewicht der Zusammensetzung, vorliegt und Natriumcocoylglutamat, sofern vorhanden, in einer Menge von 2 bis 3 Gew.-%, bezogen auf das Gewicht der Zusammensetzung, vorliegt;
wobei das Glykolipid, sofern vorhanden, in einer Menge von 3 bis 7 Gew.-%, bezogen auf das Gewicht der Zusammensetzung, vorliegt; und
wobei die Zusammensetzung frei von Natriumlaurylsulfat ist.

2. Zusammensetzung nach Anspruch 1, wobei das Glykolipid Rhamnolipid ist.

3. Zusammensetzung nach einem der vorstehenden Ansprüche, wobei das Tensidsystem ferner ein zwitterionisches Betain-Tensid und ein nichtionisches Blockcopolymer umfasst.

4. Zusammensetzung nach Anspruch 3, wobei das zwitterionische Betain-Tensid in einer Menge von 1 bis 1,5 Gew.-% der Zusammensetzung und das nichtionische Blockcopolymer in einer Menge von 0,3 bis 0,7 Gew.-% der Zusammensetzung vorliegt.

5. Zusammensetzung nach Anspruch 3 oder Anspruch 4, wobei das zwitterionische Betain-Tensid Cocamidopropylbetain ist und das nichtionische Blockcopolymer ein Poly(propylenoxid)/Poly(ethylenoxid)-Copolymer ist.

6. Zusammensetzung nach einem der vorstehenden Ansprüche, wobei die Zinkionenquelle ausgewählt ist aus der Gruppe, bestehend aus Zinkoxid, Zinksulfat, Zinkchlorid, Zinkcitrat, Zinklactat, Zinkgluconat, Zinkmalat, Zinktartrat, Zinkcarbonat, Zinkphosphat und einer Kombination davon.

7. Zusammensetzung nach Anspruch 6, wobei die Zinkionenquelle ausgewählt ist aus der Gruppe, bestehend aus Zinkoxid, Zinkcitrat und einer Kombination davon.

8. Zusammensetzung nach Anspruch 7, wobei die Zusammensetzung Zinkoxid in einer Menge von 0,5 bis 2 Gew.-% der Zusammensetzung und Zinkcitrat in einer Menge von 0,1 bis 1 Gew.-% der Zusammensetzung umfasst.

9. Zusammensetzung nach einem der vorstehenden Ansprüche, wobei die Zusammensetzung eine Fluoridionenquelle umfasst.

10. Zusammensetzung nach einem der vorstehenden Ansprüche, wobei die Zusammensetzung ein Verdickungsmittel umfasst, wobei optional das Verdickungsmittel Xanthan und Carboxymethylcellulose umfasst.

11. Zusammensetzung nach einem der vorstehenden Ansprüche, wobei die Zusammensetzung ein Silica-Abrasiv umfasst.

12. Mundpflegezusammensetzung nach einem der vorstehenden Ansprüche, wobei die Mundpflegezusammensetzung eine Zahnpasta ist.

## Revendications

1. Composition de soins bucco-dentaires comprenant un acide aminé basique sous forme libre ou sous forme de sel, dans laquelle l'acide aminé basique comprend de l'arginine et est présent en une quantité de 1 % à 15 % en poids de la composition, calculée sous forme de base libre ;
une source d'ions zinc et
un système tensioactif comprenant du lauryl glucoside et du cocoyl glutamate de sodium ; ou un glycolipide ;
dans laquelle le lauryl glucoside, s'il est présent, est présent en une quantité de 2 % à 3 % en poids de la composition et le cocoyl glutamate de sodium, s'il est présent, est présent en une quantité de 2 % à 3 % en poids de la composition ;
dans laquelle le glycolipide, s'il est présent, est présent en une quantité de 3 % à 7 % en poids de la composition ; et
dans laquelle la composition est exempte de laurylsulfate de sodium.

2. Composition selon la revendication 1, dans laquelle le glycolipide est un rham-nolipide.

3. Composition selon l'une quelconque des revendications précédentes, dans laquelle le système tensioactif comprend en outre un tensioactif zwitterionique de type bétaïne et un copolymère séquencé non ionique.

4. Composition selon la revendication 3, dans laquelle le tensioactif zwitterionique de type bétaïne est présent en une quantité de 1 % à 1,5 % en poids de la composition et le copolymère séquencé non ionique est présent en une quantité de 0,3 % à 0,7 % en poids de la composition.

5. Composition selon la revendication 3 ou la revendication 4, dans laquelle le tensioactif zwitterionique de type bétaïne est la cocamidopropylbétaïne et le copolymère séquencé non ionique est un copolymère de poly(oxyde de propylène)/poly(oxyde d'éthylène).

6. Composition selon l'une quelconque des revendications précédentes, dans laquelle la source d'ions zinc est choisie dans le groupe constitué par l'oxyde de zinc, le sulfate de zinc, le chlorure de zinc, le citrate de zinc, le lactate de zinc, le gluconate de zinc, le malate de zinc, le tartrate de zinc, le carbonate de zinc, le phosphate de zinc et une combinaison de ceux-ci.

7. Composition selon la revendication 6, dans laquelle la source d'ions zinc est choisie dans le groupe constitué par l'oxyde de zinc, le citrate de zinc et une combinaison de ceux-ci.

8. Composition selon la revendication 7, dans laquelle la composition comprend de l'oxyde de zinc en une quantité de 0,5 % à 2 % en poids de la composition et du citrate de zinc en une quantité de 0,1 % à 1 % en poids de la composition.

9. Composition selon l'une quelconque des revendications précédentes, dans laquelle la composition comprend une source d'ions fluorure.

10. Composition selon l'une quelconque des revendications précédentes, dans laquelle la composition comprend un agent épaississant, éventuellement dans laquelle l'agent épaississant comprend de la gomme de xanthane et de la carboxymé-thylcellulose.

11. Composition selon l'une quelconque des revendications précédentes, dans laquelle la composition comprend un abrasif à base de silice.

12. Composition de soins bucco-dentaires selon l'une quelconque des revendications précédentes, dans laquelle la composition de soins bucco-dentaires est un dentifrice.
